# EUROPEAN PATENT SPECIFICATION

(11) **EP 2 205 296 B1**
(45) Date of publication and mention of the grant of the patent: **31.07.2013**
(21) Application number: 07827669.8
(22) Date of filing: 05.09.2007
(51) Int. Cl.: A61M 1/36

(54) **IMPROVED PROTECTION FILTER FOR HEMODIALYSIS LINES**
VERBESSERTER SCHUTZFILTER FÜR HÄMODIALYSELEITUNGEN
FILTRE DE PROTECTION AMÉLIORÉ DESTINÉ À DES LIGNES D'HÉMODIALYSE

(43) Date of publication of application: 14.07.2010
(73) Proprietor: GVS S.p.A., 40069 Zola Predosa (Bologna) (IT)
(72) Inventor: SCAGLIARINI, Massimo, I-40033 Casalecchio di Reno (Bologna) (IT); REGGIANI, Simone, I-40133 Bologna (IT)
(74) Representative: Ripamonti, Enrico
(86) International application number: PCT/IT2007/000615
(87) International publication number: WO 2009/031171

(56) References cited:
- EP-A- 1 016 443
- WO-A-2006/000859
- DE-A1- 2 651 342
- DE-A1- 2 837 058
- US-A- 5 271 880

## Description

The present invention relates to a protection filter for hemodialysis lines in accordance with the introduction to the main claim and as described in WO-A-2006/000859.

A protection filter for hemodialysis lines is known to comprise a body connected to two conduits, of which a first conduit is connected to a dialysis machine and the second conduit is directed towards the patient. The body of known filters usually presents a normally discoidal central portion having opposing faces from which usually cylindrical or frusto-conical connection members project for connection to said conduits. Each connection member or coupling is provided with a through hole opening at the free end of the relative member and joined to the other hole within the central portion of the filter body. In this latter, between the two holes a filter element is present generally defined by an antibacterial, hydrophobic and homophobic membrane - i.e. a media filter system.

The purpose of this membrane is to enable air to be transferred from one conduit to the other via a filter (blocking the blood) in order to enable, via this latter, the hemodialysis treatment to be controlled while being carried out on a patient and to preserve (or protect) the dialysis machine from possible contact with the blood. This control is in fact achieved by measuring the pressure of the hemodialysis line by passage of the air passing through the filter, this air generating a pressure in the conduit directed towards the dialysis machine, this pressure being measured by known usual sensor means positioned in that conduit or in the machine. The filter element (membrane or other) hence enables air to pass while blocking blood flow towards the machine, the patient dialysis process being controlled by means of this passage.

Known filters, of any form, present a body generally produced in two or more parts welded together (for example by ultrasonic welding). For example a known filter comprises a body presenting two parts, each provided with a generally discoidal portion, from a face of which there projects a member for coupling or connecting to a relative conduit (tube). The filter is produced by coupling together the generally discoidal portions of the two parts after inserting a filter element between them; the two coupled parts of the two portions are then welded together, to define the filter body. EP1016443, DE 2837058 and DE 2651342 describe some known filters of the above cited kind.

This known solution therefore presents an undeniable production complexity, which reflects on the filter production costs.

Another known process for producing a protection filter for hemodialysis lines comprises firstly moulding from plastic a part of the filter body presenting the discoidal portion and the coupling member, then opening the mould to arrange the filter element on the discoidal portion and then overmoulding the second part of the filter body provided with the other discoidal portion and the relative coupling member. This method of producing the filter for hemodialysis lines results in a non-homogeneous body in which its two parts are separated and distinguishable from each other. This solution is also laborious and costly.

Moreover as the known solutions comprise a plurality of operations to be carried out on the individual components of the filter body, they inevitably lead to a non-negligible percentage of product defects , this also having to be considered in evaluating the final cost of the correctly finished product ready for sale. These defects can also be problematic if they are not identified prior to marketing the filter in that they can lead for example to blood passage through the filter towards the dialysis machine.

An object of the present invention is to provide a protection filter for hemodialysis lines which overcomes the drawbacks of the known solutions and which represents an improvement thereon.

Another object s to provide a protection filter for hemodialysis lines of the aforestated type which is of simpler production than known filters and is hence of lower production cost then these.

A further object is to provide a filter of the aforestated type which offers considerable safety in use.

These and other objects which will be apparent to the expert of the art are attained by a protection filter for hemodialysis lines in accordance with the accompanying claims.

The present invention will be more apparent from the accompanying drawing, which is provided by way of non-limiting example and in which:
Figure 1 is a partly broken-away perspective view of a first embodiment of the invention; and

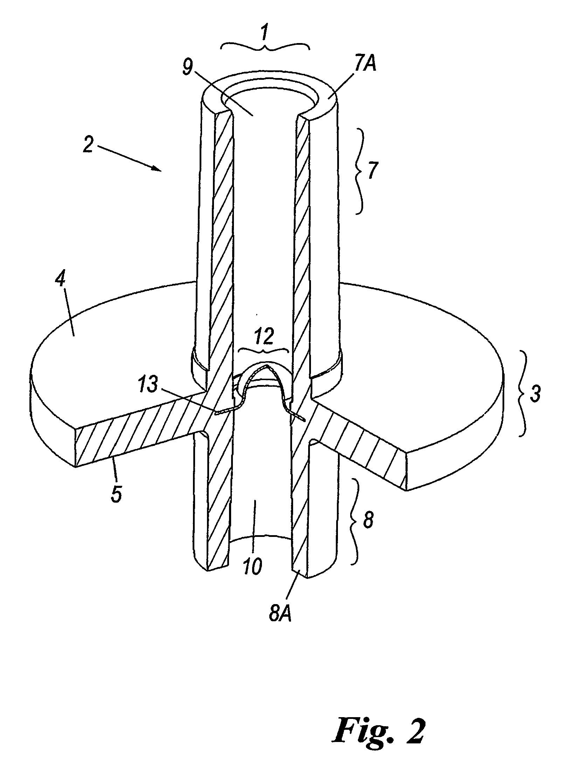

Figure 2 is a perspective view similar to that of Figure 1, but of a different embodiment of the invention.

With reference to said figures, a protection filter for hemodialysis lines is indicated overall by 1 and presents a completely homogeneous one-piece body with no joining lines or regions in its various parts. In the embodiments shown in the figures by way of non-limiting example, these parts comprise a substantially central discoidal portion 3 useful for "manipulating" the filter, and having two opposing flat faces 4 and 6 with coupling members 7 and 8 projecting from said faces 4, 5. These members are arranged to cooperate with conduits or tubes directed towards a patient subjected to dialysis and towards a dialysis machine. The coupling members comprise a through hole 9, 10 respectively, opening at the free ends 7A, 8A of the corresponding member 7, 8. The holes 9 and 10 are for example (but not necessarily) coaxial and are connected together at the discoidal portion 3 where they are separated by a filter element 12.

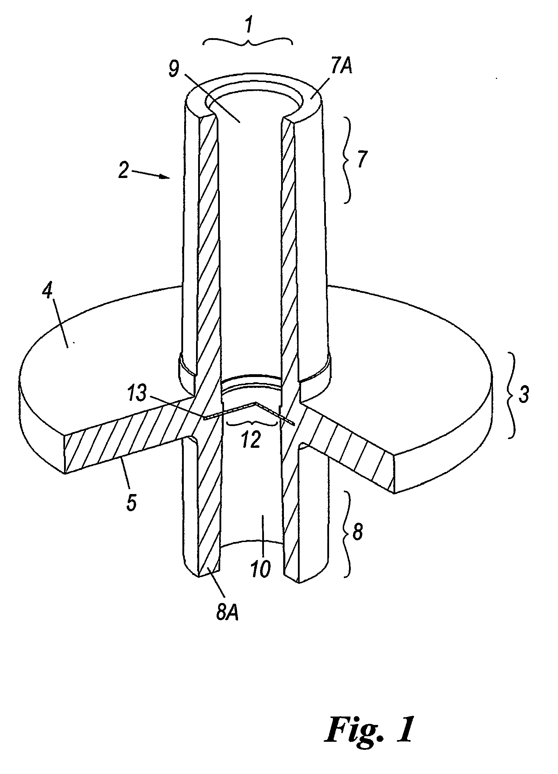

The filter element 12 presents at least one end edge 13 incorporated into the one-piece body 2 so that it becomes securely fixed to this latter. This element can be flat (Figure 1) or of beak type with pleated or folded walls or it can extend axially within the body 2 and present a plurality of surface pleats which increase the transfer surface. As an alternative, the element 12 can be of hemispherical form similar to a dome or have any other form able to define a large filtering surface for equal bulk.

The discoidal portion can advantageously present a raised end edge rising peripherally from one of its faces 4, 5, said edge facilitating manipulation of the filter in connecting the body 2 to at least one corresponding conduit. The body 2 of the filter 1, produced by known plastic moulding, is of very small size. For example it has an axial height between 10 and 50 mm and a diameter between 4 and 20 mm. The portion 3 can have a diameter which depends mainly on its appearance and related to the positioning of the filter in the dialysis machine, and is between 4 and 50 mm. The body is produced by moulding in a single operation (method known as overmoulding or insert moulding) during which the members 7 and 8 and the portion 3 are formed simultaneously, said elements being overmoulded simultaneously on the filter element 12, which is hence incorporated and inserted into the body 2 of the filter 1.

The invention is hence of simple and rapid implementation. Being in one piece and being without joining lines between its various components (the members 7, 8 and the portion 3), it is also of reliable use in that its shape ensures lack of seepage and leakages in terms of blood and air. The body 2 of the filter 1 is produced in a single moulding operation and is hence of much lower complexity and costs than similar known solutions. A particular embodiment of the invention has been described. However the body 2 of the filter 1 can also be of different shape than that shown in the drawings; for example, it can be completely tubular, without the generally discoidal portion 3 (with flat faces or with a raised perimetral edge), in which case the members 7 and 8 are defined by the ends of the tubular body. In another embodiment, this body can be prismatic and provided with the members 7 and 8. In any event, whatever its shape this body is in one piece and without joining lines, it being provided with a through conduit defined by holes provided in the coupling members 7 and 8 and mutually communicating via a filter element in the body interior and with its lateral edge incorporated into said filter body.

These solutions are also to be considered as falling within the scope of the following claims.

## Claims

1. A protection filter for hemodialysis lines (1) comprising a body (2) provided with conduit coupling members (7, 8), a first conduit being connected to a dialysis machine and the second conduit being directed towards a patient, said coupling members (7, 8) presenting internal holes (9, 10) opening at their ends (7A, 8A), said holes (9, 10) mutually communicating within the interior of said body (2), within this latter a filter element (12) being present disposed between the holes (9, 10) of the coupling members (7, 8), said body (2) incorporating the end edges (13) of the filter element (12), **characterised in that** the body (2) of said filter (1) is in one piece, is homogeneous and is without joining lines between its various parts (3, 7, 8).

2. A protection filter for hemodialysis lines as claimed in claim 1, **characterised by** being of plastic material moulded in a single moulding operation.

3. A protection filter for hemodialysis lines as claimed in claim 2, **characterised in that** the moulding is an overmoulding or an insert moulding.

4. A protection filter for hemodialysis lines as claimed in claim 1, **characterised in that** the filter element (12) is flat.

5. A protection filter for hemodialysis lines as claimed in claim 1, **characterised in that** the filter element (12) is of the type axially elongated within the body (2) along the interior of one of the coaxial holes (9, 10) present in one of its coupling members.

6. A protection filter for hemodialysis lines as claimed in claim 5, **characterised in that** the filter element (12) has folded or pleated walls.

7. A protection filter for hemodialysis lines as claimed in claim 5, **characterised in that** the filter element (12) has a hemispherical wall.

8. A protection filter for hemodialysis lines as claimed in claim 1, **characterised in that** the internal holes (9, 10) of the coupling members (7, 8) are coaxial.

9. A protection filter for hemodialysis lines as claimed in claim 1, **characterised in that** the body (2) is tubular, its opposing ends defining the coupling members (7, 8).

10. A protection filter for hemodialysis lines as claimed in claim 1, **characterised in that** the body (2) is prismatic.

11. A protection filter for hemodialysis lines as claimed in claim 1, **characterised in that** the body (2) presents a substantially discoidal laterally projecting portion (3).

12. A protection filter for hemodialysis lines as claimed in claim 11, **characterised in that** said portion comprises two opposing faces (4, 5) from which the coupling members (7, 8) project.

13. A protection filter for hemodialysis lines as claimed in claim 12, **characterised in that** one of the opposing faces (4, 5) of the substantially central portion (3) presents a raised free edge.

14. A protection filter for hemodialysis lines as claimed in claim 1, **characterised by** having an axial height of between 10 and 50 mm and a diameter of between 4 and 50 mm.

15. A method for producing a protection filter for hemodialysis lines claimed in claim 1, **characterised by** comprising a single moulding operation effected with plastic material on a filter element (12) inserted into a mould, said single moulding operation resulting in a homogeneous one-piece filter body (2) without joining lines between the various parts (3, 7, 8), said body incorporating the end edges (13) of the filter element (12).

## Patentansprüche

1. Ein Schutzfilter für Hämodialyseleitungen (1), der einen Körper (2) umfasst, welcher mit entsprechenden Leitungskupplungselementen (7, 8) ausgerüstet ist, wobei eine erste Leitung mit einer Dialysemaschine verbunden ist und eine zweite Leitung in Richtung des jeweiligen Patienten geführt wird, und die genannten Kupplungselemente (7, 8) innere Öffnungen (9, 10) aufweisen, die sich jeweils an deren Enden (7A, 8A) öffnen, wobei die genannten Öffnungen (9, 10) im Inneren des genannten Körpers (2) miteinander kommunizieren, und in diesem letzteren ein Filterelement (12) vorhanden ist, das zwischen den Öffnungen (9, 10) der Kupplungselemente (7, 8) angeordnet ist, wobei der genannte Körper (2) jeweils die Endkanten (13) des entsprechenden Filterelements (12) einbezieht, **dadurch gekennzeichnet, dass** der Körper (2) des genannten Filters (1) einteilig, gleichförmig und ohne Verbindungsnähte zwischen den einzelnen Teilen ist (3, 7, 8) ist.

2. Ein Schutzfilter für Hämodialyseleitungen gemäß Anspruch 1, **dadurch gekennzeichnet, dass** er aus Kunststoffmaterial besteht, das in einem einzigen Formungsvorgang geformt wird.

3. Ein Schutzfilter für Hämodialyseleitungen gemäß Anspruch 2, **dadurch gekennzeichnet, dass** es sich bei der Formung um ein Überspritzen oder Hinterspritzen handelt.

4. Ein Schutzfilter für Hämodialyseleitungen gemäß Anspruch 1, **dadurch gekennzeichnet, dass** das Filterelement (12) flach ist.

5. Ein Schutzfilter für Hämodialyseleitungen gemäß Anspruch 1, **dadurch gekennzeichnet, dass** das Filterelement (12) des im Körper (2) axial verlängerten Typs ist, und zwar am Inneren einer der koaxialen Öffnungen (9, 10) entlang, welche sich in einem seiner Kupplungselemente befinden.

6. Ein Schutzfilter für Hämodialyseleitungen gemäß Anspruch 5, **dadurch gekennzeichnet, dass** das Filterelement (12) jeweils gefalzte oder gefaltete Wände aufweist.

7. Ein Schutzfilter für Hämodialyseleitungen gemäß Anspruch 5, **dadurch gekennzeichnet, dass** das Filterelement (12) eine hemisphärische Wand aufweist.

8. Ein Schutzfilter für Hämodialyseleitungen gemäß Anspruch 1, **dadurch gekennzeichnet, dass** die inneren Öffnungen (9, 10) der Kupplungselemente (7, 8) koaxial sind.

9. Ein Schutzfilter für Hämodialyseleitungen gemäß Anspruch 1, **dadurch gekennzeichnet, dass** der Körper (2) röhrenförmig ist, wobei seine gegenüberliegenden Enden jeweils die Kupplungselemente (7, 8) bilden.

10. Ein Schutzfilter für Hämodialyseleitungen gemäß Anspruch 1, **dadurch gekennzeichnet, dass** der Körper (2) prismatisch ist.

11. Ein Schutzfilter für Hämodialyseleitungen gemäß Anspruch 1, **dadurch gekennzeichnet, dass** der Körper (2) einen im wesentlichen scheibenförmigen, seitlich vorstehenden Abschnitt (3) aufweist.

12. Ein Schutzfilter für Hämodialyseleitungen gemäß Anspruch 11, **dadurch gekennzeichnet, dass** der genannte Abschnitt jeweils zwei gegenüberliegende Seiten (4, 5) umfasst, von denen die Kupplungselemente (7, 8) vorstehen.

13. Ein Schutzfilter für Hämodialyseleitungen gemäß Anspruch 12, **dadurch gekennzeichnet, dass** eine der gegenüberliegenden Seiten (4, 5) des im wesentlichen zentrischen Abschnitts (3) eine erhöhte freie Kante aufweist.

14. Ein Schutzfilter für Hämodialyseleitungen gemäß Anspruch 1, **dadurch gekennzeichnet, dass** er eine axiale Höhe aufweist, die zwischen 10 und 50 mm liegt, sowie einen Durchmesser zwischen 4 und 50 mm.

15. Eine Methode zur Herstellung eines Schutzfilters für Hämodialyseleitungen gemäß Anspruch 1, **dadurch gekennzeichnet, dass** sie einen einzigen Formungsvorgang umfasst, der mit Kunststoffmaterial an einem Filterelement (12) erfolgt, das in eine Form eingesetzt wird, wobei der genannte einzige Formungsvorgang einen gleichförmigen, einteiligen Filterkörper (2) ohne Verbindungsnähte zwischen den einzelnen Teilen (3, 7, 8) ergibt und der genannte Körper dabei jeweils die Endkanten (13) des entsprechenden Filterelements (12) einbezieht.

## Revendications

1. Filtre de protection destiné à des lignes d'hémodialyse (1) qui comprend un corps (2) pourvu d'éléments de raccordement de conduit (7, 8), un premier conduit étant relié à un dialyseur et le second conduit étant dirigé vers un patient, lesdits éléments de raccordement (7, 8) présentant des cavités internes (9, 10) s'ouvrant à leurs extrémités (7A, 8A), lesdites cavités (9, 10) communiquant mutuellement au sein de l'intérieur dudit corps (2), un élément filtre (12), présent au sein dudit corps, étant disposé entre les cavités (9, 10) des éléments de raccordement (7, 8), le corps (2) incorporant les bords d'extrémité (13) de l'élément filtre (12), **caractérisé en ce que** le corps (2) dudit filtre (1) est réalisé en une seule pièce, est homogène et ne présente pas de lignes de jonction entre ses différentes parties (3, 7, 8).

2. Filtre de protection destiné à des lignes d'hémodialyse selon la revendication 1, **caractérisé en ce qu'**il est réalisé en matériau plastique moulé au moyen d'une seule opération de moulage.

3. Filtre de protection destiné à des lignes d'hémodialyse selon la revendication 2, **caractérisé en ce que** le moulage est un surmoulage ou un moulage par injection.

4. Filtre de protection destiné à des lignes d'hémodialyse selon la revendication 1, **caractérisé en ce que** l'élément filtre (12) est plat.

5. Filtre de protection destiné à des lignes d'hémodialyse selon la revendication 1, **caractérisé en ce que** l'élément filtre (12) est de type allongé dans le sens axial au sein du corps (2) le long de l'intérieur d'une des cavités coaxiales (9, 10) présentes dans un de ses éléments de raccordement.

6. Filtre de protection destiné à des lignes d'hémodialyse selon la revendication 5, **caractérisé en ce que** l'élément filtre (12) présente des parois plissées ou pliées.

7. Filtre de protection destiné à des lignes d'hémodialyse selon la revendication 5, **caractérisé en ce que** l'élément filtre (12) présente une paroi hémisphérique.

8. Filtre de protection destiné à des lignes d'hémodialyse selon la revendication 1, **caractérisé en ce que** les cavités internes (9, 10) des éléments de raccordement (7, 8) sont coaxiales.

9. Filtre de protection destiné à des lignes d'hémodialyse selon la revendication 1, **caractérisé en ce que** le corps (2) est tubulaire, ses extrémités opposées définissant les éléments de raccordement (7, 8).

10. Filtre de protection destiné à des lignes d'hémodialyse selon la revendication 1, **caractérisé en ce que** le corps (2) est réalisé en forme de prisme.

11. Filtre de protection destiné à des lignes d'hémodialyse selon la revendication 1, **caractérisé en ce que** le corps (2) présente une partie sortant latéralement en saillie substantiellement discoïdale (3).

12. Filtre de protection destiné à des lignes d'hémodialyse selon la revendication 11, **caractérisé en ce que** ladite partie comprend deux faces opposées (4, 5) à partir desquelles les éléments de raccordement (7, 8) sortent en saillie.

13. Filtre de protection destiné à des lignes d'hémodialyse selon la revendication 12, **caractérisé en ce que** l'une des faces opposées (4, 5) de la partie substantiellement centrale (3) présente un bord libre ourlé.

14. Filtre de protection destiné à des lignes d'hémodialyse selon la revendication 1, **caractérisé en ce qu'**il présente une hauteur axiale comprise entre 10 et 50 mm et un diamètre compris entre 4 et 50 mm.

15. Méthode pour la production d'un filtre de protection destiné à des lignes d'hémodialyse selon la revendication 1, **caractérisé en ce qu'**il comprend une seule opération de moulage réalisée à l'aide d'un matériau plastique sur un élément filtre (12) introduit dans un moule, ladite seule opération de moulage résultant en un corps de filtre homogène d'une seule pièce (2) ne présentant pas de lignes de jonction entre les différentes parties (3, 7, 8), ledit corps incorporant les bords d'extrémité (13) de l'élément filtre (12).
